# EUROPEAN PATENT APPLICATION

(11) **EP 3 747 504 A1**
(43) Date of publication of application: **09.12.2020**
(21) Application number: 19178331.5
(22) Date of filing: 05.06.2019
(51) Int. Cl.: A61N 1/372, A61N 1/375

(54) **ANTENNA FOR AN IMPLANTABLE PULSE GENERATOR**

(71) Applicant: GTX medical B.V., 5656 AE Eindhoven (NL)
(72) Inventor: Pflug, Hans, 5656 AE Eindhoven (NL); Tol, Jeroen, 5656 AE Eindhoven (NL); Koen, Weijand, 03580 l' Alfàs del Pi, Alicante (ES)
(74) Representative: DTS Patent- und Rechtsanwälte Schnekenbühl und Partner mbB

(57) **Abstract**

An antenna (10) for an implantable pulse generator (IPG), the antenna (10) being configured for resonant inductive wireless power transfer and/or near field magnetic induction communication, the antenna (10) comprising at least two sets (12a, 12b) each comprising several windings (14a, 14b).

Furthermore, the present invention relates to an implantable pulse generator (IPG).

## Description

The present invention relates to an antenna for a pulse generating system, in particular for a communication and powering system of a pulse generating system.

Pulse generating systems are known from various medical applications, inter alia from pacemakers and neuromodulation applications, such as neuromodulation for the treatment of a subject, e.g. in the field of improving recovery after neurological disorders such as spinal cord injury and/or stroke.

In particular, such pulse generation systems often are implantable pulse generating systems and are used in systems to deliver adaptive electrical spinal cord stimulation to facilitate and restore locomotion after neuromotor impairment as e.g. described in EP 2 868 343 A1.

US 7 813 809 B2 describes an implantable pulse generator (IPG) for prosthetic or therapeutic stimulation of muscles, nerves, or central nervous system tissue, or any combination is sized and configured to be implanted in subcutaneous tissue. The IPG includes a case and a control circuitry located within the case, and includes a primary cell or rechargeable power source, a receive coil for receiving an RF magnetic field to recharge the rechargeable power source, non-inductive wireless telemetry circuitry, and a microcontroller for control of the IPG.

Communication and power charging systems in a medical field are known from the prior art and especially used to submit energy and communication signals transcutaneously, in particular between a non-implanted device and an IPG.

For example, US 2015/0012061 A1 relates to a medical device for providing a stimulation therapy including a call configurator to receive both inductive charging and telemetric signals. Inductive charging signals are in a first frequency band. The telemetric signals are in a second frequency band higher than the first frequency band. The medical device includes inductive charging circuitry configured to provide electrical power to the medical device via the inductive charging signals. The medical device includes telemetric circuitry configured to conduct telecommunications with the external device via the telemetric signals. The medical device includes a first component electrically coupled between the coil and the inductive charging circuitry. The first component is configured to allow the inductive charging signals to pass through. The medical device includes a second component electrically coupled between the coil and the telemetric circuitry. The second component is configured to substantially drop the inductive charging signals while allowing telemetric signals to pass through.

Alternative solutions are for example disclosed by EP 1 680 182 A1, EP 1 675 648 A1 and EP 1 575 665 A1.

Of note, IPG coils are usually attached to the exterior of the housing of the IPG or placed in the housing, in particular the main housing. The first approach results in a larger size of the IPG in terms of its largest width and thus is an adverse option, as the larger the size of the IPG is, the worse it is for the patient. The second approach is also an adverse option due to the conducting properties of the usually used titanium housing.

Preferably, the coil could be placed in an IPG header which is typically made of nonconducting material and attached to the IPG housing or IPG main housing. This probably represents the most practical solution of integrating a coil into an IPG, both in terms of form factor of the IPG and in terms of IPG performance. However, this includes the risk of implanting the IPG with the side of the header containing the coil not closest to the skin of a patient, resulting in an a-symmetric header coil, in terms of implantation depth of the IPG header coil. This may adversely increase the implantation depth of the header coil by half the header thickness and thus reduce performance of the coil and/or the IPG. In other words, the distance between the IPG (header) coil and the coil of the related non-implanted device sending signals to the IPG is not optimal.

It is an object of the present invention to improve an antenna and/or an induction coil of an IPG of a neuromodulation and/or neurostimulation system, in particular in that a better performance of the IPG in terms of increasing a coupling coefficient or coupling factor and therefore the neuromodulation and/or neurostimulation system is enabled.

This object is solved according to the present invention with an antenna with the features of claim 1. Accordingly, an antenna for an implantable pulse generator (IPG), the antenna being configured for resonant inductive wireless power transfer and/or near field magnetic induction communication, the antenna comprising at least two sets each comprising several windings.

The invention is based on the basic idea that, by designing an antenna for an IPG, in particular for an IPG for neuromodulation and/or neurostimulation, comprising at least two sets each comprising several windings, one can avoid that the distance between coil and skin is not minimal. Thus, it does not matter which side of the IPG, in particular the header, faces the skin of the patient and thus is closer to the related non-implanted device. In other words, the features of the antenna according to the present invention enable placing the antenna in minimal distance to the skin of the patient, thus in minimal distance for resonant inductive wireless power transfer and/or near field magnetic induction communication (NFMI). The antenna design is therefore optimized for transferring a required amount of current from a non-implanted device to the IPG, in particular into an IPG battery to charge the battery. Further, the antenna design is optimized for communication between a non-implanted device and the IPG (bidirectionally, using NFMI). Overall, this enables better performance of the IPG and optimized neuromodulation/ neurostimulation.

In general, the antenna may be or may comprise at least one coil.

Further, a set comprising several windings may comprise at least one coil.

An IPG antenna and/or coil may be used for 5-8 MHz, especially 6.78 MHz, resonant inductive wireless power transfer and 8-12 MHz, especially 10.6 MHz, NFMI communication.

In particular, the at least two sets of windings may comprise an identical number of windings. By using at least two sets of windings comprising an identical number of windings an absolutely symmetrical design of the antenna may be enabled. As a consequence, it may be avoided that the distance between the antenna and the skin of the patient is not minimal. Further as a consequence, this may enable best possible signal and/or power transfer independent of how the IPG is implanted in terms of direction.

The number of windings may be four or more. In particular, inductance results inter alia from the number of windings of a coil. Due to the magnetic concatenation of the individual windings, caused by the spatially close arrangement of the individual windings, the inductance of wound coils theoretically increases in square with the number of windings. A doubling of the number of windings with the same geometric dimensions results in a quadrupling of the inductance. Thus, by using four or more windings a certain level of inductance may be enabled.

Further, the at least two sets of windings each may form a rectangular form, especially a trapezoidal form. Beyond the number of windings, the inductance of a coil also results from the dimension of a coil. Given that the housing of IPGs or certain parts of IPGs, such as a header are often characterized by a rectangular form, especially a trapezoidal form, the at least two sets of windings forming a rectangular form, especially a trapezoidal form may enable maximum use of the dimension of the housing of the IPG. Thus, using the at least two sets of windings each forming a rectangular form, especially a trapezoidal form may enable a maximum level of inductance relative to the given dimensions of the housing of the IPG.

Further, the at least two sets of windings may be connected by means of at least one bridging element. This connection between the at least two sets of windings may enable that the inductance of a first set of windings may be combined with the inductance of a second set of windings. In other words, the current flowing in one set of windings may induce a voltage in the adjacent, second set of windings, finally causing an increased mutual inductance of the at least two sets comprising several windings.

According to the present invention, an IPG comprising at least one antenna as described above is disclosed.

In particular, the IPG may have a main housing part and a header part attached to the housing, wherein the at least one antenna may be arranged in the header part. As mentioned above, this arrangement results in the most practical solution of integrating a coil into an IPG in terms of form factor of the IPG, available assembly space and in terms of IPG performance.

An IPG antenna and/or coil may be used for 5-8 MHz, especially 6.78 MHz, resonant inductive wireless power transfer and 8-12 MHz, especially 10.6 MHz, near field magnetic induction communication. Arranging the antenna in the metal housing of an IPG, such as titanium, is disadvantageous when using the antenna and/or coil for 5-8 MHz, especially 6.78 MHz, resonant inductive wireless power transfer and 8-12 MHz, especially 10.6 MHz, near field magnetic induction communication, due to the conducting properties of the housing leading in eddy-current losses. Thus, arranging the IPG antenna and/or coil in the titanium housing part of the IPG is not desirable. Further, arranging the antenna around the housing of the IPG is disadvantageous and not desirable as this may result in a larger size of the IPG in terms of its largest width. Arranging the antenna in the header part may overcome these disadvantages.

Further, the header part may be transparent. By using a transparent header part, it may be enabled that the integrity of the sets of windings may be checked visually before implanting the IPG for quality control reasons for example.

In particular, the header part may be made of plastic. In particular, the header part may be made of medical grade polyamide and/or medical grade polypropylene. Alternatively, and/or additionally, the header may be at least partially made of medial grade silicone. As the header may be made of non-conductive material it may not support or function as an indifferent electrode due to eddy-current losses, leading to better performance of the IPG.

Further, the header made of plastic may have insulation effects.

The at least two sets each comprising several windings may be arranged in parallel. In particular, arranging the first set comprising several windings and the second set comprising several windings in parallel may enable maximal total (mutual) inductance.

Further, the antenna may comprise at least one further set of several windings, wherein the at least one further set of several windings may be arranged orthogonally with regard to the at least two sets each comprising several windings. In particular, the at least one further set of several windings may increase the coupling factor between the antenna and/or coil of the implanted IPG and an antenna and/or coil of a non-implanted device which needs to charge the IPG battery or communicate with the IPG for controlling reasons. During communication, the non-implanted device may be in a non-optimal orientation with regard to the at least two sets each comprising several windings arranged in parallel, resulting in a low coupling factor, e.g. coupling factor null. By introducing at least one further set comprising several windings orthogonally with regard to the at least two sets each comprising several windings in the IPG header, communication quality and performance may be improved for these non-optimal orientations without impacting the charging performance.

The IPG may comprise connector slots being arranged at least partially within the at least one antenna.

Generally, an IPG header may include one or more connector-slots, and the feedthroughs are typically located in the center of the housing-side. Mounting the connectors in the header center may make the header construction easier. According to the present invention, this construction advantage may be maintained with the connector slots being arranged at least partially within the at least one antenna.

Further, the connection of the connector slots with the housing-feedthroughs may be more cumbersome in case the connectors are placed more towards one side of the header (outside of its center). This may require bending of the connector connections towards the feedthroughs, therefore requiring more header height. Therefore, placing the connectors at least partially within the at least one antenna, e.g. in the middle of the antenna, and having the antenna next to the connectors on both sides may lead to a lower volume claim of the antenna in the header and/or the header itself.

Further details and advantages of the present invention shall now be disclosed in connection with the drawings.

It is shown in
- Fig. 1: a schematic perspective view of a first embodiment of an antenna together with an implantable pulse generator according to the present invention;
- Fig. 2a: a schematic top view of the first embodiment of the antenna together with the implantable pulse generator according to Fig. 1 in more/greater detail;
- Fig. 2b: a schematic side view of the first embodiment of the antenna together with the implantable pulse generator according to Fig. 1 in more/greater detail;
- Fig. 3a: a diagram of a coil coupling coefficient of the implanted antenna together with the implantable pulse generator according to Fig. 1; and
- Fig. 3b: a further diagram of a coil coupling coefficient of the implanted antenna together with the implantable pulse generator according to Fig. 1.

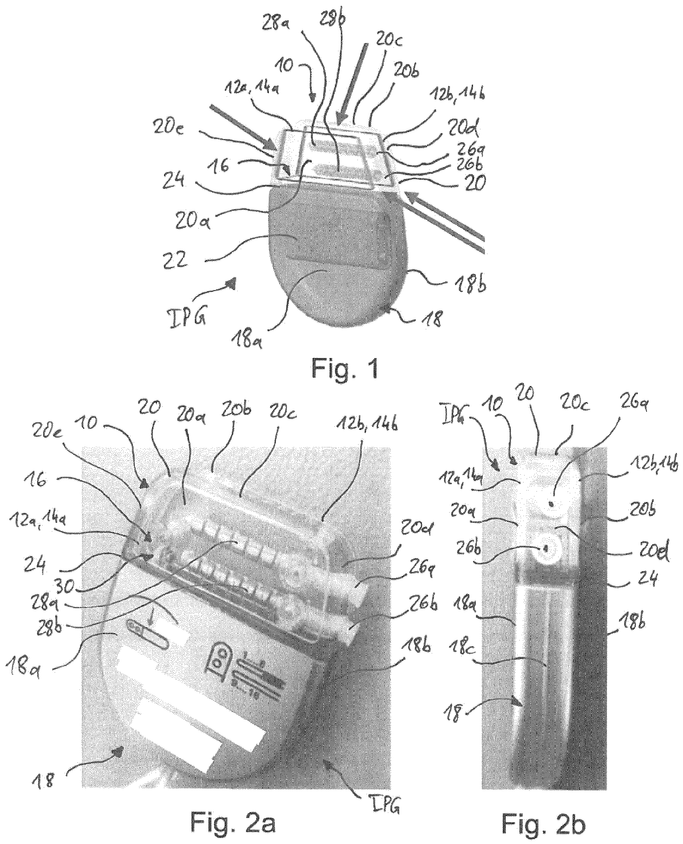

**Fig. 1** shows a schematic perspective view of a first embodiment of an antenna 10 together with an implantable pulse generator IPG according to the present invention.

The antenna 10 comprises two sets 12a, 12b, wherein each set 12a, 12b comprises several windings 14a, 14b.

The number of windings 14a, 14b of each set is four.

According to a further embodiment, the number of windings 14a, 14b of each set 12a, 12b is an integer being bigger than four.

The two sets 12a, 12b of windings 14a, 14b comprise an identical number of windings 14a, 14b.

The two sets 12a, 12b form an induction-coil having an overall number of eight windings 14a, 14b (or so-called turns).

Thus, the two sets 12a, 12b of windings 14a, 14b are connected by means of a bridging element 16.

Each of the two sets 12a, 12b of windings 14a, 14b form a rectangular form.

Especially, each of the two sets 12a, 12b of windings 14a, 14b form a trapezoidal form.

Further, Fig. 1 shows an implantable pulse generator IPG comprising the antenna 10 as mentioned above.

The implantable pulse generator IPG has a main housing part 18.

The main housing part 18 is formed of a medical-grade metal alloy such as titanium alloys Ti-6Al-4V-ELI or TAV-ELI.

The main housing part 18 constitutes a thin-walled and shell-like housing structure which surrounds the inner components of the implantable pulse generator IPG in a fluid tight sealed manner.

Most important inner components are the battery 22 and the control electronics (not shown in Fig. 1) coupled thereto for generating specific stimulation patterns for neuromodulation, especially neurostimulation, after spinal cord injuries and/or stroke as known in the prior art.

On the top face (based on an implanted condition) of the main housing part 18, which is arranged opposite to its bottom, a header part 20 is attached to the main housing part 18.

According to Fig. 1, the antenna 10 is arranged in the header part 20.

At the top face of the main housing part 18, this main housing part 18 and the header part 20 form, at a corresponding sealing face, a further fluid tight sealing with an elastomeric O-Ring 24 having a rectangular shape.

The header part 20 has a substantially trapezoidal form.

The header part 20 consists of a first and second main surface 20a, 20b (each with the biggest surface area of the header part 20) arranged in parallel.

Further, the first and second main surface 20a, 20b flush with the two planar outer surfaces 18a, 18b of the main housing part 18 being also arranged in parallel with regard to each other.

The header part 20 further comprises one header part top surface 20c being orientated opposite of the joint sealing face formed by the main housing part 18 and the header part 20.

The header part 20 also comprises two side surfaces 20d, 20e extending between the sealing face and the header part top surface 20c on the one hand.

On the other hand, these two side surfaces 20d, 20e also extend between the two main surfaces 20a, 20b.

The two sets 12a, 12b of the windings 14a, 14b are arranged at the first and second main surfaces 20a, 20b of the header part 20.

Consequently, the two sets 12a, 12b of the windings 14a, 14b form a symmetrical arrangement within the header part 20 (as indicated by the two parallel arrows).

Especially, the two sets 12a, 12b of the windings 14a, 14b form a parallel arrangement within the header part 20.

The two additional arrows of Fig. 1 (shown in a crooked fashion) indicate further optional locations of additional third and fourth sets of windings.

This third set of windings is arranged at one side surface 20d, 20e (as indicated by the corresponding arrow).

Alternatively, the third set of windings is arranged at both of side surfaces 20d, 20e.

Additionally, or alternatively, the fourth set of windings is arranged at the header part top surface 20c.

These additional third and fourth sets of windings each have a substantially orthogonal orientation with respect to the first and second sets 12a, 12b of windings 14a, 14b.

These additional sets of windings improve the coil coupling between the motion controller (not shown in Fig. 1) and the implantable pulse generator IPG in case of a non-optimal orientation between the motion controller and the implantable pulse generator IPG.

The third and/or fourth sets of windings is/are built up in the same manner as the first and second sets 12a, 12b of windings 14a, 14b.

As can be further depicted from Fig. 1, the header part 20 is transparent and made of plastic.

The plastic is made of medical grade plastic such as medical grade polyamide or medical grade polypropylene.

The plastic may alternatively be made of medical grade silicone.

**Fig. 2a** shows a schematic top view of the first embodiment of the antenna 10 together with the implantable pulse generator IPG according to Fig. 1 in more detail.

Especially, the two sets 12a, 12b of the windings 14a, 14b are arranged at the edges of the first and second main surfaces 20a, 20b of the header part 20.

For example, the two sets 12a, 12b of the windings 14a, 14b are arranged at the edges of the first and second main surfaces 20a, 20b of the header part 20 by embedding them therein.

The embedding can be done as here for example shown by overmolding. Alternatively, injection molding is also possible.

Further, the optional third and/or fourth sets of windings is/are integrated in the side and header part top surface(s) 20c, 20d, 20e in the same manner as described for the main surfaces 20a, 20b.

As can be depicted in greater detail in Fig. 2a, the two sets 12a, 12b of windings 14a, 14b are connected by a bridging element 16.

The bridging element 16 may be optionally also used for further connection of the third and fourth sets of windings to the two sets 12a, 12b of windings 14a, 14b.

This bridging element 16 is also embedded within the header part 20 and extends between the two sets 12a, 12b of windings 14a, 14b at one or both side surfaces 20d, 20e.

As can be further depicted from Fig. 2a, the implantable pulse generator IPG, at its header part 20 comprises a first and second connector slot 26a, 26b for connecting the two corresponding lead connectors 28a, 28b with the two control leads of the spinal cord stimulation paddle (not shown in Fig. 2a).

These connector slots 26a, 26b are arranged partially or completely within the antenna 10, i.e. within the projected area formed by the first and second sets 12a, 12b of the windings 14a, 14b.

Additionally, the two sets 12a, 12b of windings 14a, 14b, the connector slots 26a, 26b, and the corresponding lead connectors 28a, 28b form a sandwich-like structure with the connector slots 26a, 26b and the corresponding lead connectors 28a, 28b disposed in between the two sets 12a, 12b of windings 14a, 14b.

Especially, the connector slots 26a, 26b are arranged in one of the side surfaces 20d, 20e of the header part 20.

This arrangement causes the third set of windings to be arranged at the opposite side surface 20d, 20e of the side surface 20d, 20e with the connector slots 26a, 26b disposed therein.

The two sets 12a, 12b of windings 14a, 14b (or optionally the third and/or fourth sets) are further connected with the control electronics and the battery 22 (each not shown in Fig. 2a) inside the main housing part 18 via a connection area 30 serving as a connection interface.

Furthermore, a plurality of connecting wires connecting each terminal of the two lead connectors 28a, 28b to the corresponding pins of the feed-through capacitors of control electronics are also shown in greater detail in Fig. 2a.

**Fig. 2b** shows a schematic side view of the first embodiment of the antenna 10 together with the implantable pulse generator IPG according to Fig. 1 in more detail.

Especially, the individual windings 14a, 14b of the first and second set 12a, 12b may be depicted in more detail.

Additionally, a weld 18c in the center of the main housing part 18 is visible therein as the main housing part 18 is built up by welding two housing part shells together.

Additionally, the function of the antenna 10 as shown in Figs. 1 to 2b together with the implantable pulse generator IPG is as follows:
The antenna 10 for an implantable pulse generator IPG is configured for 6.78 MHz resonant inductive wireless power transfer and for 10.6 MHz near field magnetic induction communication.

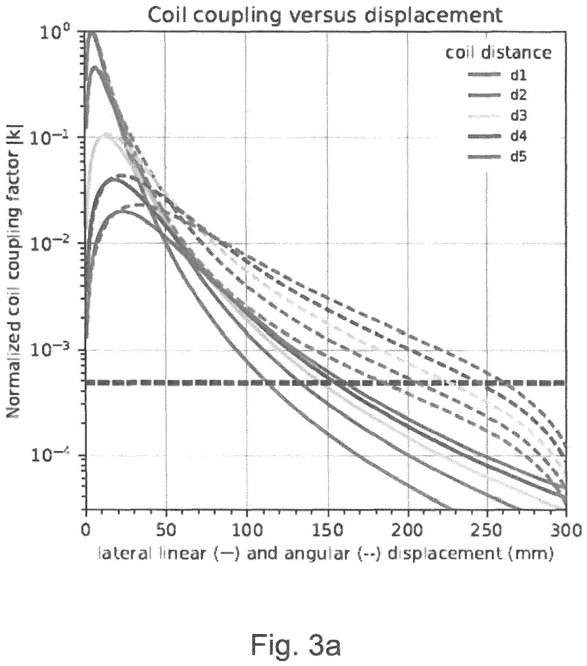

In this context, **Fig. 3a** shows a diagram of a coil coupling coefficient or factor k of the implanted antenna 10 together with the implantable pulse generator IPG according to Fig. 1 to Fig. 2b.

The coil coupling coefficient k is shown between an external and non-implanted coil (e.g. a charging or communication coil) and the implanted antenna 10 together with the implantable pulse generator IPG versus a lateral displacement (both linear and in an angular fashion around a round body model) of the antenna 10.

This graph corresponds to a communication use-case, showing (as a dashed horizontal line) the coupling factor k corresponding to a receiver sensitivity level, assuming a specific known transmitter output level.

In Fig. 3a, the coupling factor k of the antenna 10 is shown without additional orthogonal coils or sets of windings (e.g. at the side surface 20d, 20e and/or header top surface 20c), showing an improvement in k-factor compared to the prior art.

The non-implanted coil for communication is a different one, compared to the used wireless charging coil.

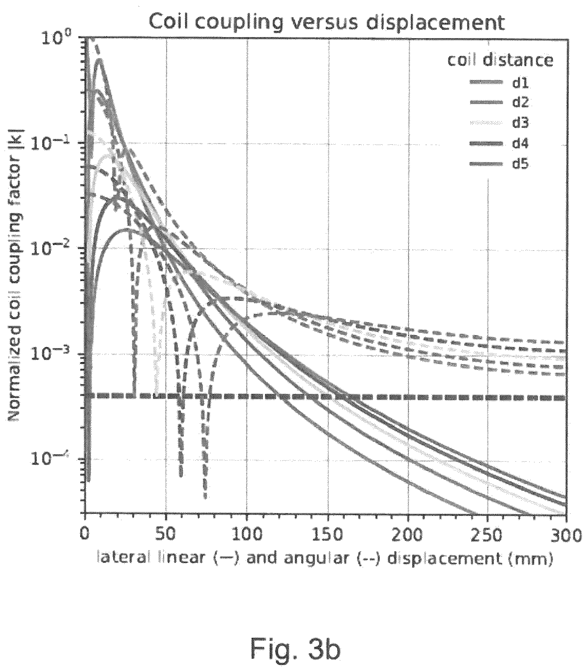

**Fig. 3b** shows, in contrast to Fig. 3a, a further diagram of a coil coupling coefficient k of the implanted antenna of the implantable pulse generator according to Fig. 1 to Fig. 2b.

Compared to the set up as described in Fig. 3a, the following modifications of the antenna 10 have been implemented:
In contrast to Fig. 3a, Fig. 3b shows the coupling factor k of the antenna 10 with at least one additional orthogonal set of windings (see Fig. 1, e.g. at the side surface 20d, 20e and/or header top surface 20c), showing an improvement in the k-factor, especially for the angular case (dashed line), leading to a larger communication range.

Further, the charging performance is not meaningfully impacted by the added orthogonal set(s) of windings in the header 20.

Thus, the header antenna 10 (sets 12a, 12b of windings 14a, 14b at the main surfaces 20a, 20b) may still be used for both charging and communication.

### References

- 10: antenna
- 12a: set of windings
- 12b: set of windings
- 14a: windings
- 14b: windings
- 16: bridging element
- 18: main housing part
- 18a: planar outer surface
- 18b: planar outer surface
- 18c: weld
- 20: header part
- 20a: first main surface
- 20b: second main surface
- 20c: header part top surface
- 20d: side surface
- 20e: side surface
- 22: battery
- 24: O-Ring
- 26a: connector slot
- 26b: connector slot
- 28a: lead connector
- 28b: lead connector
- 30: connection area

- IPG: implantable pulse generator

## Claims

1. An antenna (10) for an implantable pulse generator (IPG), the antenna (10) being configured for resonant inductive wireless power transfer and/or near field magnetic induction communication, the antenna (10) comprising at least two sets (12a, 12b) each comprising several windings (14a, 14b).

2. The antenna (10) according to claim 1, **characterized in that** the at least two sets (12a, 12b) of windings (14a, 14b) comprise an identical number of windings (14a, 14b).

3. The antenna (10) according to claim 1 or 2, **characterized in that** the number of windings (14a, 14b) is four or more.

4. The antenna (10) according to claim 1, 2, or 3, **characterized in that** the at least two sets (12a, 12b) of windings (14a, 14b) each form a rectangular form, especially a trapezoidal form.

5. The antenna (10) according to any of the preceding claims, **characterized in that** the at least two sets (12a, 12b) of windings (14a, 14b) are connected by means of a least one bridging element (16).

6. An implantable pulse generator (IPG) comprising at least one antenna (10) according to one of the claims 1 to 5.

7. The implantable pulse generator (IPG) according to claim 6, **characterized in that** the implantable pulse generator (IPG) has a main housing part (18) and a header part (20) attached to the main housing part (18), wherein the at least one antenna (10) is arranged in the header part (20).

8. The implantable pulse generator (IPG) according to claim 6 or 7, **characterized in that** the header part (20) is transparent.

9. The implantable pulse generator (IPG) according to one of the claims 6 to 8, **characterized in that** the header part (20) is made of plastic.

10. The implantable pulse generator (IPG) according to one of the claims 6 to 9, **characterized in that** the at least two sets (12a, 12b) each comprising several windings (14a, 14b) are arranged in parallel.

11. The implantable pulse generator (IPG) according to one of the claims 6 to 10, **characterized in that** the antenna (10) comprises at least one further set of several windings, wherein the at least one further set of several windings is arranged orthogonally with regard to the at least two sets (12a, 12b) each comprising several windings (14a, 14b).

12. The implantable pulse generator (IPG) according to one of claims 6 to 11, **characterized in that** the implantable pulse generator (IPG) comprises connector slots being arranged at least partially within the at least one antenna.
